# EUROPÄISCHE PATENTSCHRIFT

(11) **EP 2 907 469 B1**
(45) Veröffentlichungstag und Bekanntmachung des Hinweises auf die Patenterteilung: **07.04.2021**
(21) Anmeldenummer: 15155080.3
(22) Anmeldetag: 13.02.2015
(51) Int. Cl.: B62B 3/10, A61B 50/10

(54) **Gerätewagen**
Device trolley
Servante d'outillage

(30) Priorität: 18.02.2014 DE 102014101997
(43) Veröffentlichungstag der Anmeldung: 19.08.2015
(73) Patentinhaber: Karl Storz SE & Co. KG, 78532 Tuttlingen (DE)
(72) Erfinder: Sabo, Alexander, 78532 Tuttlingen (DE); Jansche, Andreas, 78532 Tuttlingen (DE); Gümpel, Anika, 78532 Tuttlingen (DE); Müller-Beiter, Wolfgang, 72517 Sigmaringendorf (DE); Schairer, Rainer, 72459 Albstadt (DE)

(56) Entgegenhaltungen:
- DE-U- 1 825 620
- FR-A- 1 200 640
- US-A- 2 973 233
- US-A- 4 743 040
- US-A- 5 035 332
- US-A1- 2011 040 242

## Beschreibung

Die vorliegende Erfindung ist auf einen Geräteträger, insbesondere auf einen zerlegbaren, modifizierbaren und auch von technisch nicht ausgebildeten Personen fehlerfrei zusammensetzbaren Geräteträger bezogen, wobei der Geräteträger insbesondere ein Gerätewagen ist.

In Operationssälen und anderen medizinischen Behandlungsräumen kommen oft viele medizinische Geräte und Instrumente gleichzeitig oder nacheinander zum Einsatz. Ein medizinischer Geräteträger, insbesondere ein medizinischer Gerätewagen, d.h. ein medizinischer Geräteträger, der auf Rädern verfahrbar ist, kann eine ökonomisch und ergonomisch günstige und flexible Anordnung einer Vielzahl von medizinischen Geräten und Instrumenten ermöglichen.

Ein medizinischer Gerätewagen oder ein anderer medizinischer Geräteträger soll nicht nur robust sein und eine einfache Reinigung und Sterilisation ermöglichen, sondern auch mit geringem Aufwand modifiziert bzw. verändert (insbesondere erweitert) und, möglichst auch von Personen ohne technische Ausbildung sicher und fehlerfrei zusammengesetzt werden können.

Ein Geräteträger ist aus der US 20220402342 A bekannt.

Eine Aufgabe der vorliegenden Erfindung besteht darin, einen verbesserten Geräteträger zu schaffen, insbesondere einen Geräteträger, der auch von nicht technisch ausgebildetem Personal fehlerfrei zusammengesetzt werden kann.

Diese Aufgabe wird durch den Gegenstand des Anspruchs 1 gelöst.

Weiterbildungen sind in den abhängigen Ansprüchen angegeben.

Ein Geräteträger umfasst ein Flächenmodul mit einer ersten Befestigungseinrichtung und ein Säulenmodul mit einer zweiten Befestigungseinrichtung, wobei die erste Befestigungseinrichtung am Flächenmodul und die zweite Befestigungseinrichtung am Säulenmodul so zueinander korrespondierend ausgebildet sind, dass das Säulenmodul und das Flächenmodul mit einer ersten, linearen Bewegung und einer nachfolgenden zweiten, rotatorischen Bewegung des Säulenmoduls relativ zum Flächenmodul miteinander verbindbar sind.

Der Geräteträger ist insbesondere ein medizinischer Geräteträger, dabei insbesondere ein medizinischer Gerätewagen bzw. ein Gerätewagen für medizinischen Geräte oder ein Gerätewagen für nicht-medizinische Anwendungen oder ein Geräteträger für stationäre medizinische oder nicht-medizinische Anwendungen. Der Geräteträger ist insbesondere zum Tragen, Bereithalten oder Vorhalten von mehreren medizinischen oder nicht-medizinischen Geräten und Instrumenten in einem Operationssaal oder einem anderen medizinischen Behandlungsraum oder in oder außerhalb eines anderen Raums ausgebildet. Dazu sind insbesondere auf dem Flächenmodul oder auf zusätzlichen Fachböden eine oder mehrere Flächen vorgesehen, auf die medizinische Geräte und Instrumente oder eine andere Nutzlast gestellt oder gelegt werden kann. Ferner kann der Geräteträger einen oder mehrere Auszüge oder durch Türen oder Klappen verschließbare Fächer aufweisen.

Das Flächenmodul ist insbesondere ein Bodenmodul, das den Geräteträger nach unten abschließt, auf dem ein oder mehrere Säulenmodule angeordnet sein können, und an dessen Unterseite Rollen vorgesehen sein können. Das Bodenmodul kann insbesondere rechteckig oder annähernd rechteckig sein. Alternativ ist das Flächenmodul ein Brückenmodul, das die Säulenmodule nicht an ihren unteren Enden, sondern an ihren oberen Enden oder an anderer Stelle verbindet. Das Flächenmodul, insbesondere das Brückenmodul, ist vorzugsweise trapezförmig oder annähernd trapezförmig ausgebildet. Das oder die Säulenmodule sind insbesondere langerstreckt und orthogonal zum Flächenmodul und bei der vorgesehenen Anordnung des Geräteträgers auf einem horizontalen Boden vertikal ausgerichtet. Bei Vorsehen eines Bodenmoduls und eines Brückenmoduls, können eines oder mehrere Säulenmodule senkrecht auf beiden Flächenmodulen und zwischen diesen angeordnet sein. Eines oder mehrere der Säulenmodule können insbesondere einen oder mehrere Hohlräume oder Kanäle zur Aufnahme von Kabeln, Verbindungsleitungen und dergleichen zum Anschluss an die vom Geräteträger getragenen Geräte aufweisen.

Die zur Verbindung des Säulenmoduls mit dem Flächenmodul zunächst erforderliche erste, lineare Bewegung erfolgt insbesondere vertikal bzw. orthogonal zum Bodenmodul und insbesondere parallel zum Säulenmodul. Die erste, lineare Bewegung ist eine lineare Bewegung bis zu der Position des Säulenmoduls relativ zum Flächenmodul, bei der die zweite, rotatorische Bewegung ausgeführt werden kann, um Säulenmodul und Flächenmodul miteinander zu verbinden. Die erste, lineare Bewegung endet bei Berührung der einander zugewandten Oberflächen des Flächenmoduls und des dem Flächenmodul zuzuwendenden Endes des Säulenmoduls. Die Verbindung erfolgt insbesondere zwischen einer seitlichen Endfläche eines langerstreckten Säulenmoduls und dem Flächenmodul.

Die nachfolgende zweite, rotatorische Bewegung des Säulenmoduls erfolgt insbesondere um eine Achse, die parallel zur Längsachse des Säulenmoduls, orthogonal zum Bodenmodul und bei der vorgesehenen Anordnung des Geräteträgers auf einem horizontalen Boden vertikal ist. Die zweite, rotatorische Bewegung umfasst insbesondere eine Rotation um einen vorbestimmten Winkel, der beispielsweise im Bereich zwischen 10 Grad und 120 Grad, insbesondere im Bereich zwischen 20 Grad und 60 Grad liegt. Nach Ausführung zunächst der ersten, linearen Bewegung und danach der zweiten, rotatorischen Bewegung ist das Säulenmodul zumindest lose mit dem Flächenmodul verbunden, d. h. die Verbindung muss noch nicht so starr und fest sein, wie für die Verwendung des Geräteträgers erforderlich. Eine feste und starre Verbindung des Säulenmoduls mit dem Flächenmodul kann darüber hinaus beispielsweise ein Anziehen bzw. Festziehen von einer oder mehreren Schrauben erfordern.

Die Kombination aus Schieben und Drehen bzw. aus einer ersten, linearen Bewegung und einer zweiten, rotatorischen Bewegung für eine zumindest lose Verbindung des Säulenmoduls mit dem Flächenmodul kann besonders für Personen ohne technische Ausbildung eine Vereinfachung darstellen. Die erste, lineare Bewegung und die zweite, rotatorische Bewegung können schnell ausgeführt werden und die anschließende Handhabung der beiden zumindest lose miteinander verbundenen Module vereinfachen.

Die zweite, rotatorische Bewegung ist insbesondere eine rein rotatorische Bewegung, die nicht, wie beispielsweise bei einer Schraubverbindung, mit einer weiteren linearen Bewegung einhergeht.

Bei einem Geräteträger, wie er hier beschrieben ist, umfasst die erste Befestigungseinrichtung eine Befestigungsöffnung oder mehrere Befestigungsöffnungen am Flächenmodul, wobei die zweite Befestigungseinrichtung einen Kopf oder mehrere Köpfe am Säulenmodul umfasst, der bzw. die gegenüber dem dem Flächenmodul zuzuwendenden Ende des Säulenmoduls überstehen und dafür vorgesehen und ausgebildet sind, um in je eine Befestigungsöffnung am Flächenmodul einzugreifen.

Bei einem Geräteträger, wie er hier beschrieben ist, umfasst die zweite Befestigungseinrichtung eine oder mehrere Befestigungsöffnungen am Säulenmodul, wobei die erste Befestigungseinrichtung einen oder mehrere Köpfe am Flächenmodul umfasst, die gegenüber der dem Säulenmodul zuzuwendenden Seite des Flächenmoduls überstehen und dafür vorgesehen und ausgebildet sein, um in je eine Befestigungsöffnung am Säulenmodul einzugreifen.

Der Kopf oder die Köpfe sind insbesondere pilzförmig oder im Wesentlichen pilzförmig oder weisen zumindest in einer Ebene orthogonal zu ihrer Bewegung bei der zweiten, rotatorischen Bewegung des Säulenmoduls relativ zum Flächenmodul einen T-förmigen oder L-förmigen Querschnitt oder einen im Wesentlichen T-förmigen oder L-förmigen Querschnitt auf. Der Kopf oder die Köpfe weisen in Ebenen orthogonal zur Richtung der ersten, linearen Bewegung und orthogonal zur Rotationsachse der zweiten, rotatorischen Bewegung insbesondere nahe demjenigen Modul, an dem sie befestigt sind, einen kleineren oder schmaleren Querschnitt auf als in größerem Abstand von diesem Modul. Der Kopf oder die Köpfe weisen insbesondere jeweils eine Gestalt ähnlich dem Kopf und einem Teil des Schafts einer Schraube auf, die nur so weit eingeschraubt ist, dass nicht nur der vollständige Kopf, sondern auch ein Teil des Schafts übersteht.

Bei einem nicht erfindungsgemäßen Geräteträger, umfasst die erste Befestigungseinrichtung insbesondere eine oder mehrere Befestigungsöffnungen am Flächenmodul, wobei die zweite Befestigungseinrichtung eine bzw. mehrere Schrauben am Säulenmodul umfasst und der Schraubenkopf der Schraube bzw. die Schraubenköpfe der Schrauben gegenüber dem dem Flächenmodul zuzuwendenden Ende des Säulenmoduls überstehen und dafür vorgesehen und ausgebildet sind, um in je eine Befestigungsöffnung am Flächenmodul einzugreifen.

Bei einem nicht erfindungsgemäßen Geräteträger, umfasst die erste Befestigungseinrichtung insbesondere eine oder mehrere Schrauben am Flächenmodul, wobei die zweite Befestigungseinrichtung eine bzw. mehrere Befestigungsöffnungen am Säulenmodul umfasst und der Schraubenkopf der Schraube bzw. die Schraubenköpfe der Schrauben gegenüber dem Flächenmodul überstehen und dafür vorgesehen und ausgebildet sind, um in je eine Befestigungsöffnung am Säulenmodul einzugreifen.

Bei einem Geräteträger, wie er hier beschrieben ist, sind die Befestigungsöffnung oder die Befestigungsöffnungen insbesondere jeweils schlüssellochförmig ausgebildet.

Die Befestigungsöffnung oder die Befestigungsöffnungen weisen insbesondere ähnlich einem altertümlichen Schlüsselloch für einen Bartschlüssel einen breiten und einen schmalen Abschnitt auf. Der breite Abschnitt der Befestigungsöffnung ist insbesondere so ausgebildet, dass der Kopf einer Zugstange mit der ersten, linearen Bewegung durch den breiten Abschnitt hindurchgeführt werden kann. Der schmale Abschnitt der Befestigungsöffnung ist insbesondere so ausgebildet, dass der Kopf mit der zweiten, rotatorischen Bewegung in den schmalen Abschnitt der Befestigungsöffnung hinein bewegt werden, jedoch dort nicht aus der Befestigungsöffnung heraus gezogen werden kann.

Der breite Abschnitt einer schlüssellochförmigen Befestigungsöffnung ist insbesondere kreisförmig. Der schmale Abschnitt einer schlüssellochförmigen Befestigungsöffnung weist insbesondere die Gestalt eines kreisbogenförmigen Streifens konstanter Breite auf, wobei das vom breiten Abschnitt der Befestigungsöffnung abgewandte Ende des schmalen Abschnitts beispielsweise halbkreisförmig ausgestaltet ist. Der Krümmungsmittelpunkt des kreisbogenförmigen Streifens bzw. der Mittelpunkt des Kreisbogens, der durch den schmalen Abschnitt definiert ist, liegt insbesondere auf der Rotationsachse der zweiten, rotatorischen Bewegung. Im Fall mehrerer schlüssellochförmiger Befestigungsöffnungen liegen insbesondere die Krümmungsmittelpunkte der schmalen Abschnitte aller Befestigungsöffnungen auf der Rotationsachse der zweiten, rotatorischen Bewegung. Im Fall gleicher Abstände von der Rotationsachse der zweiten, rotatorischen Bewegung liegen die schmalen Abschnitte aller schlüssellochförmigen Befestigungsöffnungen auf dem gleichen Kreis oder auf parallelen Kreisen gleicher Radien, dessen bzw. deren Mittelpunkte auf der Rotationsachse liegen. Im Fall unterschiedlicher Abstände von der Rotationsachse der zweiten, rotatorischen Bewegung liegen die schmalen Abschnitte aller schlüssellochförmigen Befestigungsöffnungen auf verschiedenen Kreisen (in der gleichen oder in mehreren parallelen Ebenen) mit unterschiedlichen Radien, deren Mittelpunkte auf der Rotationsachse liegen.

Bei einem erfindungsgemäßen Geräteträger, wie er hier beschrieben ist, sind die Befestigungseinrichtungen so ausgebildet und angeordnet, dass die erste, lineare Bewegung nur bei einer vorbestimmten Anordnung und Orientierung des Säulenmoduls relativ zum Flächenmodul möglich ist.

Die Befestigungseinrichtungen sind insbesondere nicht symmetrisch angeordnet, um die lineare Bewegung nur bei einer vorbestimmten Anordnung und Orientierung zu ermöglichen. Beispielsweise sind drei Köpfe und drei korrespondierende Befestigungsöffnungen nicht in der Gestalt eines gleichseitigen Dreiecks, sondern wie die Ecken eines Dreiecks mit zwei oder drei unterschiedlichen Seitenlängen angeordnet.

Ein erfindungsgemäßer Geräteträger, wie er hier beschrieben ist, umfasst ferner ein weiteres Säulenmodul, das mit dem Flächenmodul auf gleiche Weise verbindbar ist, wie das Säulenmodul.

Insbesondere erfolgt die zweite, rotatorische Bewegung jedoch beim Säulenmodul und beim weiteren Säulenmodul in entgegengesetzten Rotationsrichtungen. Insbesondere wenn, wie nachfolgend beschrieben, beide Säulenmodule durch ein weiteres Flächenmodul miteinander verbindbar sind, kann so schon die Verbindung der Säulenmodule mit dem weiteren Flächenmodul gleichzeitig die Verbindung der Säulenmodule mit dem Flächenmodul verriegeln.

Ein erfindungsgemäßer Geräteträger, wie er hier beschrieben ist, umfasst ferner ein weiteres Flächenmodul, das mit den Säulenmodulen verbindbar ist, wobei die Säulenmodule und das weitere Flächenmodul derart ausgebildet sind, dass das weitere Flächenmodul nur dann mit den Säulenmodulen verbindbar ist, wenn die Säulenmodule relativ zueinander so positioniert und orientiert sind, wie im Fall ihrer vorgesehenen Verbindung mit dem Flächenmodul.

Das weitere Flächenmodul ist insbesondere ein Brückenmodul, das die Säulenmodule an ihren vom Flächenmodul beabstandeten Enden, die bei der vorgesehenen Anordnung des Geräteträgers gleichzeitig die oberen Enden sind, miteinander verbindet. Alternativ sind das weitere Flächenmodul ein Bodenmodul im oben beschriebenen Sinne und das Flächenmodul ein Brückenmodul im oben beschriebenen Sinne.

Am weiteren Flächenmodul und an den dem weiteren Flächenmodul zuzuwendenden Enden der Säulenmodule sind insbesondere korrespondierende Befestigungseinrichtungen derart angeordnet, dass nur bei der vorgesehenen relativen Positionierung und Orientierung der beiden Säulenmodule die Verbindung zwischen dem weiteren Flächenmodul und den Säulenmodulen möglich ist. Dadurch sind die Verbindungen zwischen den Säulenmodulen und dem Flächenmodul verriegelt, wenn das weitere Flächenmodul mit den beiden Säulenmodulen verbunden wird. Es werden also erst die Säulenmodule mit dem Flächenmodul erfindungsgemäß verbunden, bevor dann anschließend das weitere Flächenmodul mit dem oder den Säulenmodulen verbunden werden kann.

Bei einem erfindungsgemäßen Geräteträger, wie er hier beschrieben ist, sind Bohrungen so an den dem weiteren Flächenmodul zuzuwendenden Enden der Säulenmodule und Befestigungsöffnungen so am weiteren Flächenmodul angeordnet, dass die Bohrungen nur dann mit den Befestigungsöffnungen zur Deckung gebracht werden können, wenn die Säulenmodule relativ zueinander so positioniert und orientiert sind, wie im Fall ihrer vorgesehenen Verbindung mit dem Flächenmodul.

In einem nicht erfindungsgemäßen Ausführungsbeispiel wird das weitere Flächenmodul insbesondere mittels Schrauben und/oder Niete und/oder Dübel und/oder Stifte, die durch die Befestigungsöffnungen hindurch und in die Bohrungen eingreifen, mit den Säulenmodulen verbunden.

Bei einem erfindungsgemäßen Geräteträger, wie er hier beschrieben ist, umfasst die erste Befestigungseinrichtung eine Befestigungsöffnung am Flächenmodul, wobei die zweite Befestigungseinrichtung eine Zugstange am Säulenmodul umfasst, wobei die Zugstange einen Kopf an dem dem Flächenmodul zuzuwendenden Ende des Säulenmoduls und ein Gewinde an dem dem weiteren Flächenmodul zuzuwendenden Ende des Säulenmoduls umfasst, wobei der Kopf der Zugstange dafür vorgesehen und ausgebildet ist, gegenüber dem dem Flächenmodul zuzuwendenden Ende des Säulenmoduls überzustehen und in eine Befestigungsöffnung am Flächenmodul einzugreifen, und wobei das Gewinde der Zugstange dafür vorgesehen und ausgebildet ist, gegenüber dem dem weiteren Flächenmodul zuzuwendenden Ende des Säulenmoduls überzustehen und in eine Befestigungsöffnung am weiteren Flächenmodul einzugreifen.

Die zweite Befestigungseinrichtung kann mehrere Zugstangen am Säulenmodul und/oder in jedem von mehreren Säulenmodulen eine oder mehrere Zugstangen umfassen. Das Gewinde an dem dem weiteren Flächenmodul zuzuwendenden Ende der Zugstange ist insbesondere ein Außengewinde. Die Befestigungsöffnung oder die Befestigungsöffnungen am Flächenmodul sind insbesondere jeweils schlüssellochförmig oder im Wesentlichen schlüssellochförmig. Durch eine geeignete, insbesondere asymmetrische Anordnung mehrerer Zugstangen und der korrespondierenden Befestigungsöffnungen kann wiederum erreicht werden, dass jedes Säulenmodul nur in einer vorgesehenen relativen Orientierung mit dem Flächenmodul und mit dem weiteren Flächenmodul verbindbar ist.

Nicht erfindungsgemäß kann jede Zugstange anstelle des Gewindes eine andere Einrichtung zur Einleitung einer Zugkraft aufweisen, beispielsweise eine umlaufende Nut, in die nach dem Hindurchführen des dem weiteren Flächenmodul zugewandten Endes der Zugstange ein Sprengring, ein Wellensicherungsring oder eine Sicherungsscheibe eingesetzt werden bzw. eingreifen kann.

Bei einem Geräteträger, wie er hier beschrieben ist, ist die Befestigungsöffnung am weiteren Flächenmodul insbesondere derart angeordnet, dass nur dann das Gewinde an der Zugstange in eine Befestigungsöffnung in dem weiteren Flächenmodul einführbar ist, wenn die Säulenmodule relativ zueinander so positioniert und orientiert sind, wie im Fall ihrer vorgesehenen Verbindung mit dem Flächenmodul.

Bei einem Geräteträger, wie er hier beschrieben ist, umfasst die zweite Befestigungseinrichtung insbesondere mehrere Zugstangen am Säulenmodul, wobei die Befestigungsöffnungen am weiteren Flächenmodul derart angeordnet sind, dass nur dann gleichzeitig die Gewinde an allen Zugstangen in je eine Befestigungsöffnung in dem weiteren Flächenmodul einführbar sind, wenn die Säulenmodule relativ zueinander so positioniert und orientiert sind, wie im Fall ihrer vorgesehenen Verbindung mit dem Flächenmodul.

Das Gewinde an der Zugstange bzw. die Gewinde an den Zugstangen sind insbesondere jeweils teilweise durch die Befestigungsöffnungen an dem weiteren Flächenmodul hindurchführbar, so dass sie bei an den Flächenmodulen anliegendem weiteren Flächenmodul gegenüber diesem weiteren Flächenmodul überstehen.

Bei einem Geräteträger, wie er hier beschrieben ist, ist insbesondere durch Aufschrauben und Festziehen einer Schraubenmutter an dem Gewinde der Zugstange das der Zugstange zugeordnete Säulenmodul gleichzeitig mit dem Flächenmodul und mit dem weiteren Flächenmodul starr verbindbar.

Ein Geräteträger, wie er hier beschrieben ist, umfasst insbesondere ferner Ausnehmungen im weiteren Flächenmodul zum Aufnehmen je eines dem weiteren Flächenmodul zuzuwendenden Endes je eines Säulenmoduls, wobei die Ausnehmungen derart angeordnet und ausgebildet sind, dass die dem weiteren Flächenmodul zuzuwendenden Enden der Säulenmodule nur dann gleichzeitig in je eine zugeordnete Ausnehmung einführbar sind, wenn die Säulenmodule relativ zueinander so positioniert und orientiert sind, wie im Fall ihrer vorgesehenen Verbindung mit dem Flächenmodul.

Die durch den Formschluss zwischen den Enden der Säulenmodule und den Ausnehmungen eindeutig definierte relative Anordnung und Orientierung der Säulenmodule kann die Verbindungen zwischen den Säulenmodulen und dem Flächenmodul verriegeln. Die Querschnitte der Ausnehmungen und die korrespondierenden Querschnitte der in die Ausnehmungen eingreifenden Enden der Säulenmodule sind insbesondere nicht symmetrisch, weisen beispielsweise keine rein kreisförmig oder elliptische Gestalt und nicht die Gestalt eines regelmäßigen Polyeders auf, um ein Einführen der Enden der Säulenmodule in die Ausnehmungen jeweils nur in einer vorgesehenen Orientierung zu ermöglichen.

Ein Geräteträger, wie er hier beschrieben ist, umfasst insbesondere ferner Räder zum Ableiten der Gewichtskraft des Geräteträgers in einen Boden, auf dem der Geräteträger steht, wobei die Räder eine reibungsarme Verschiebung des Geräteträgers auf dem Boden ermöglichen.

Ein Geräteträger, wie er hier beschrieben ist, umfasst insbesondere ferner Räder zum Ableiten der Gewichtskraft des Geräteträgers in eine oder mehrere Schienen, auf denen der Geräteträger steht, wobei die Räder eine reibungsarme Verschiebung des Geräteträgers auf dem Boden ermöglichen.

Die Räder sind insbesondere am Flächenmodul oder am weiteren Flächenmodul angeordnet. Die Räder und deren Rotationsachsen sind insbesondere jeweils um eine horizontale Achse schwenkbar, um eine Verschiebung des Geräteträgers nicht nur in einer geraden Richtungen zu ermöglichen. Durch die Räder wird der Geräteträger zum Gerätewagen.

### Kurzbeschreibung der Figuren

Nachfolgend werden Ausführungsformen anhand der beigefügten Figuren näher erläutert. Es zeigen:
- Figur 1: eine schematische Darstellung eines nicht erfindungsgemäßen medizinischen Gerätewagens;
- Figur 2: eine weitere schematische Darstellung des medizinischen Gerätewagens aus Figur 1;
- Figur 3: eine weitere schematische Darstellung des medizinischen Gerätewagens aus Figur 1;
- Figur 4: eine schematische Darstellung eines Bodenmoduls des medizinischen Gerätewagens aus den Figuren 1 bis 3;
- Figur 5: eine schematische Darstellung des Bodenmoduls aus Figur 4 und mehrerer Säulenmodule;
- Figur 6: eine weitere schematische Darstellung des Teils des Bodenmoduls und der Säulenmodule aus Figur 5;
- Figur 7: eine weitere schematische Darstellung des medizinischen Gerätewagens aus den Figuren 1 bis 3;
- Figur 8: eine weitere schematische Darstellung des medizinischen Gerätewagens aus den Figuren 1 bis 3 und 7;
- Figur 9: eine schematische Schnittdarstellung eines Teils eines weiteren medizinischen Gerätewagens;
- Figur 10: eine weitere schematische Darstellung des Teils des weiteren medizinischen Gerätewagens aus Figur 9;
- Figur 11: eine schematische Darstellung eines erfindungsgemäßen medizinischen Gerätewagens.

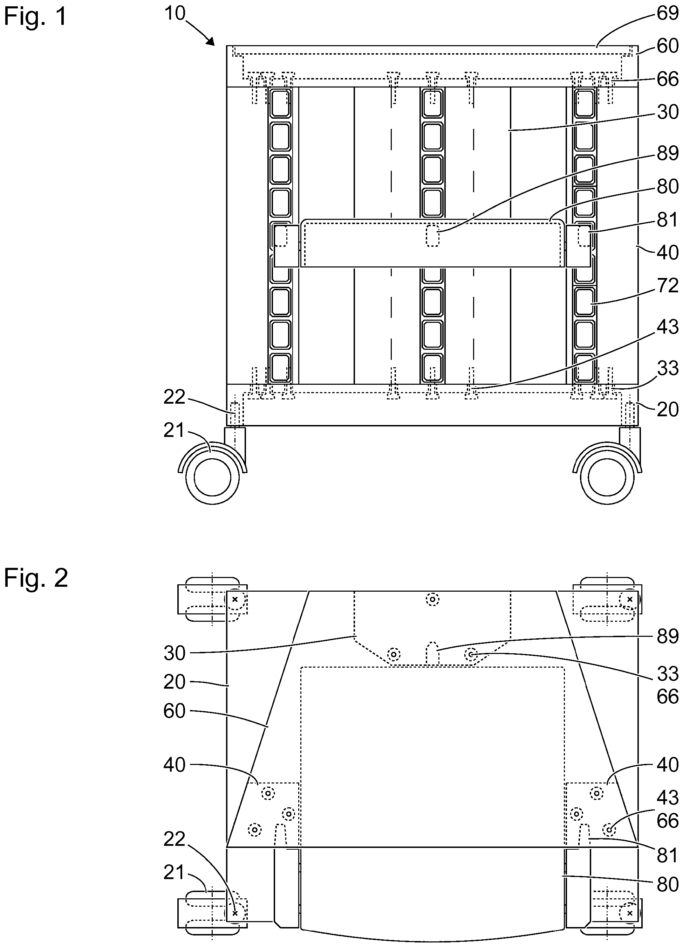

Figur 1 zeigt eine schematische Darstellung eines nicht erfindungsgemäßen medizinischen Geräteträgers, insbesondere eines medizinischen Gerätewagens 10. Die Zeichenebene der Figur 1 ist vertikal bzw. bei der vorgesehenen Verwendung des medizinischen Gerätewagens 10 orthogonal zu einer horizontalen Bodenfläche, auf der der medizinische Gerätewagen 10 steht.

Der medizinische Gerätewagen 10 umfasst mehrere Flächenmodule, nämlich ein Bodenmodul 20, ein Brückenmodul 60 und einen Fachboden 80, sowie mehrere Säulenmodule 30, 40. Die Flächenmodule 20, 60, 80 erstrecken sich im Wesentlichen parallel zur Bodenfläche, auf der der medizinische Gerätewagen 10 steht, und damit orthogonal zur Zeichenebene der Figur 1. Die Säulenmodule 30, 40 erstrecken sich im Wesentlichen orthogonal zu den Flächenmodulen 20, 60, 80 und parallel zur Zeichenebene der Figur 1.

Das Bodenmodul 20 ist bei der vorgesehenen Verwendung des medizinischen Gerätewagens 10 unten angeordnet. An der Unterseite des im Wesentlichen rechteckigen Bodenmoduls 20 ist an jeder Ecke des Bodenmoduls 20 eine Rolleneinheit 21 angeordnet. Jede Rolleneinheit 21 umfasst insbesondere zwei Rollen, mindestens jedoch eine Rolle, und ist um eine vertikale Schwenkachse 22 schwenkbar, so dass der medizinische Gerätewagen reibungsarm in beliebige Richtungen geschoben und rotiert werden kann. Das Brückenmodul 60 bildet bei dem dargestellten Beispiel den oberen Abschluss des medizinischen Gerätewagens 10.

Der medizinische Gerätewagen 10 weist eine Vorderseite auf, die dafür vorgesehen ist, medizinischem Personal zugewandt zu sein, und die bei der Darstellung in Figur 1 dem Betrachter zugewandt ist. Die zur Vorderseite entgegengesetzte Rückseite des medizinischen Gerätewagens ist bei der Darstellung in Figur 1 vom Betrachter abgewandt. Von der Vorderseite aus kann der Fachboden 80 in den medizinischen Gerätewagen 10 eingesetzt werden, und von der Vorderseite aus sind auf oder in dem medizinischen Geräteträger 10 abgestellte oder gelagerte oder bereitgehaltene medizinische Geräte oder Instrumente zugänglich.

Ein erstes Säulenmodul 30 ist an der Rückseite des medizinischen Gerätewagens 10 mittig angeordnet. Die zweiten Säulenmodule 40 sind symmetrisch zueinander an den Seiten des medizinischen Gerätewagens 10 nahe der Vorderseite des medizinischen Gerätewagens 10 angeordnet. Jedes Säulenmodul 30, 40 weist eine Mehrzahl von Öffnungen 72 auf. Die Öffnungen 72 sind an jedem Säulenmodul 30, 40 in einem regelmäßigen Raster angeordnet und jeweils in Richtung zur Vorderseite des medizinischen Gerätewagens 10 hin offen.

Der Fachboden 80 weist einen oder mehrere Stifte 89 und zwei oder mehr Eingreifhaken 81 auf, die den Öffnungen 72 zugewandt und zum Eingreifen in je eine Öffnung 72 vorgesehen und ausgebildet sind. Entsprechend sind der oder die Stifte 89 und die Eingreifhaken 81 bei der Darstellung in Figur 1 vom Betrachter abgewandt und von anderen Teilen des Fachbodens 80 verdeckt und deshalb in Figur 1 lediglich in gestrichelter Kontur angedeutet. Der oder die Stifte 89 sind mittig oder nahe der Mitte an der hinteren Kante des Fachbodens 80 angeordnet, um in je eine Öffnung 72 im ersten Säulenmodul 30 einzugreifen. Abweichend von der Darstellung in Figur 1 kann das erste Säulenmodul 30 zwei oder mehr vertikale Reihen von Öffnungen 72 aufweisen. Die Eingreifhaken 81 sind vorgesehen, angeordnet und ausgebildet, um in je eine Öffnung 72 in den seitlich angeordneten zweiten Säulenmodulen 40 einzugreifen.

Der Fachboden 80 kann in verschiedenen Höhen bzw. in verschiedenen Abständen vom Bodenmodul 20 und vom Brückenmodul 60 angeordnet werden. An einem oder an beiden Eingreifhaken 81 und/oder am Stift 89 sind in Figur 1 nicht dargestellte Einrichtungen zum Verriegeln des Fachbodens 80 an den Säulenmodulen 30, 40 vorgesehen, die in Figur 1 nicht dargestellt sind. Insbesondere werden ein oder beide Eingreifhaken 81 und/oder der Stift 89 jeweils in einer Öffnung 72 verriegelt, so dass ein unbeabsichtigtes Lösen des Fachbodens 80 aus seiner vorgesehenen Position und ein nachfolgendes Herabfallen verhindert werden.

Die Säulenmodule 30, 40 sind mittels Schrauben 33, 43 mit dem Bodenmodul 20 und mittels Schrauben 66 mit dem Brückenmodul 60 starr, jedoch lösbar verbunden. Die Schrauben 33, 43, 66 sind von außen nicht sichtbar und deshalb in Figur 1 in gestrichelten Linien dargestellt. Ferner sind in Figur 1 in gestrichelten Linien die inneren Konturen der Querschnitte des Bodenmoduls 20 und des Brückenmoduls 60 angedeutet. Bei dem dargestellten Beispiel sind die Schrauben 33, 43 zwischen dem Bodenmodul 20 und den Säulenmodulen 30, 40 von der Unterseite des medizinischen Gerätewagens 10 aus zugänglich und die Schrauben 66 zwischen dem Brückenmodul 60 und den Säulenmodulen 30, 40 durch einen Wartungsdeckel 69 verdeckt.

Der medizinische Gerätewagen 10 ist einfach, insbesondere auch durch Personen ohne technische Ausbildung, zusammensetzbar und zerlegbar. Ferner ist der medizinische Gerätewagen 10 durch Austausch der Säulenmodule 30, 40 und/oder durch Austausch des Bodenmoduls 20 und/oder durch Austausch des Brückenmoduls 60 modifizierbar. Ferner kann der Fachboden 80 durch ein oder mehrere Module mit Auszügen und/oder offenen oder durch Klappen oder Türen verschließbaren Fächern und/oder durch weitere Fachböden 80 ersetzt oder ergänzt werden. Bei der dargestellten Konfiguration können insbesondere auf dem Bodenmodul 20 und auf dem Fachboden 80 und optional zusätzlich auf dem Brückenmodul 60 medizinische Geräte, medizinische Instrumente und/oder andere Nutzlast angeordnet werden.

Figur 2 zeigt eine weitere schematische Darstellung des medizinischen Gerätewagens 10 aus Figur 1. Figur 2 zeigt eine Draufsicht, d.h. die Zeichenebene der Figur 2 ist horizontal bzw. parallel zu einer Bodenfläche, auf der der medizinische Gerätewagen 10 in seiner vorgesehenen Anordnung steht, und damit orthogonal zur Zeichenebene der Figur 1. Die bei der Darstellung in Figur 1 vom Betrachter abgewandte Rückseite des medizinischen Gerätewagens 10 liegt in Figur 2 oben, die bei der Darstellung in Figur 1 dem Betrachter zugewandte Vorderseite des medizinischen Gerätewagens 10 liegt bei der Darstellung in Figur 2 unten.

In Figur 2 ist die rechteckige Grundform des Bodenmoduls 20 mit vier Rolleneinheiten 21 an den Ecken erkennbar. Die vertikalen Schwenkachsen 22 der Rolleneinheiten 21 sind orthogonal zur Zeichenebene der Figur 2.

Über dem Bodenmodul 20 erstrecken sich die Säulenmodule 30, 40 im Wesentlichen orthogonal zur Zeichenebene der Figur 2. Jedes Säulenmodul 30, 40 weist einen im Wesentlichen polygonalen Querschnitt auf. Das erste Säulenmodul 30 ist mittig an der Rückseite des medizinischen Gerätewagens angeordnet. Die zweiten Säulenmodule 40 sind symmetrisch zueinander ausgebildet und symmetrisch zueinander an den Seiten des medizinischen Gerätewagens 10 angeordnet. Bei dem dargestellten Beispiel sind die zweiten Säulenmodule 40 näher an der Vorderseite als an der Rückseite des medizinischen Gerätewagens 10 angeordnet. Die Säulenmodule 30, 40 sind bei der Blickrichtung der Figur 2 durch das Brückenmodul 60 verdeckt. Deshalb sind in Figur 2 lediglich die äußeren Konturen der Querschnitte der Säulenmodule 30, 40 in gestrichelten Linien angedeutet.

Das Brückenmodul 60 weist bei dem dargestellten Beispiel eine im Wesentlichen trapezförmige Grundform auf und verdeckt bei der Blickrichtung der Figur 2 einen Teil des Fachbodens 80, der deshalb lediglich in gestrichelten Linien angedeutet ist. Der Wartungsdeckel 69 (vgl. Figur 1) und entsprechende Konturen an der Oberseite des Brückenmoduls 60 sind nicht gezeigt.

Der Fachboden 80 weist an beiden Seiten symmetrisch zueinander angeordnete Eingreifhaken 81 auf, die in korrespondierende Öffnungen 72 (vgl. Figur 1) in den zweiten Säulenmodulen 40 eingreifen. Ferner weist der Fachboden 80 einen Stift 89 auf, der in eine korrespondierende Öffnung 72 (vgl. Figur 1) im ersten Säulenmodul 30 eingreift.

In Figur 2 sind ferner die Schrauben 33, 43, 66 zur Verbindung der Säulenmodule 30, 40 mit dem Bodenmodul 20 und mit dem Brückenmodul 60 in gestrichelten Linien angedeutet. Bei dem dargestellten Beispiel sind die Schrauben 33, 43 zur Verbindung des Bodenmoduls 20 mit den Säulenmodulen 30, 40 einerseits und die Schraube 66 zur Verbindung des Brückenmoduls 60 mit den Säulenmodulen 30, 40 anderseits jeweils paarweise exakt vertikal übereinander und damit in der Blickrichtung der Figur 2 deckungsgleich angeordnet. Eine derartige Anordnung der Schrauben 33, 43, 66 kann beispielsweise bei einer Ausgestaltung der Säulenmodule 30, 40 aus Strangpressprofilen, die durchgehende Kanäle aufweisen, vorteilhaft sein. In diesem Fall werden die Schrauben 33, 43, 66 insbesondere in die Enden der Kanäle eingeschraubt.

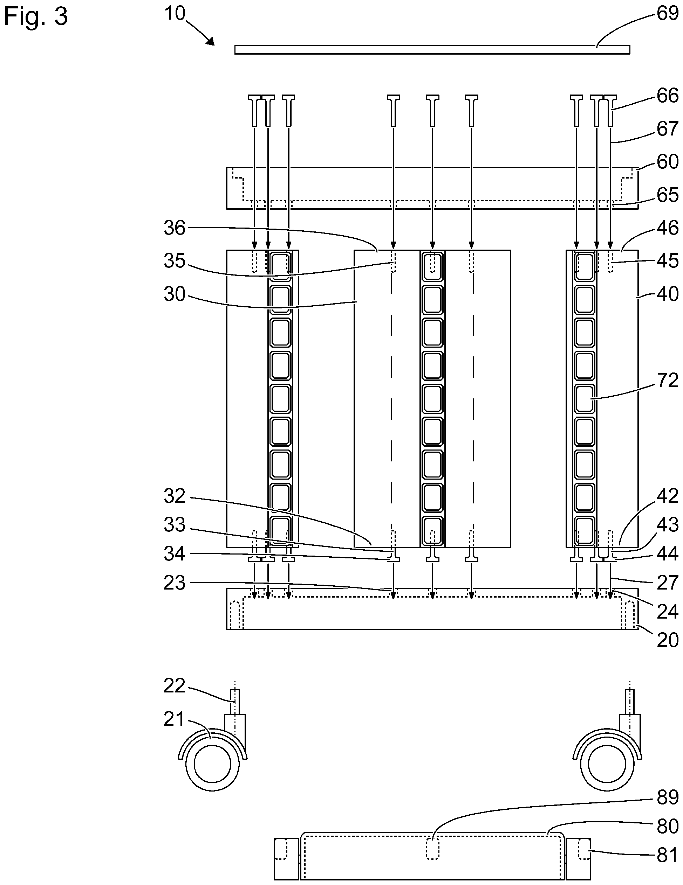

Figur 3 zeigt eine weitere schematische Darstellung des medizinischen Gerätewagens 10 aus den Figuren 1 und 2. Die Orientierung der Zeichenebene entspricht derjenigen der Figur 1. Im Unterschied zur Figur 1 sind in Figur 3 die Bestandteile des medizinischen Gerätewagens 10 in der Art einer Explosionszeichnung voneinander beabstandet, jedoch in der vorgesehenen relativen Orientierung dargestellt.

Der medizinische Gerätewagen 10 kann insbesondere in die in Figur 3 angedeutete Module bzw. Baugruppen zerlegt transportiert und an ein Krankenhaus oder eine Arztpraxis geliefert werden. Die Rolleneinheiten 21 sind beispielsweise in der von Bürostühlen bekannten Art an der Unterseite des Bodenmoduls 20 einsetzbar.

Die Schrauben 33, 43 sind insbesondere bereits bei Anlieferung an den dem Bodenmodul 20 zuzuwendenden Enden 32, 42 der Säulenmodule 30, 40 in entsprechende Bohrungen eingesetzt bzw. eingeschraubt. Dabei ist der Überstand der Köpfe 34, 44 der Schrauben 33, 43 gegenüber den dem Bodenmodul 20 zuzuwendenden Enden 32, 42 der Säulenmodule 30, 40 an die Wandstärke des Bodenmoduls 20 in der Umgebung von für die Schrauben 33, 43 vorgesehenen Befestigungsöffnungen 23, 24 angepasst.

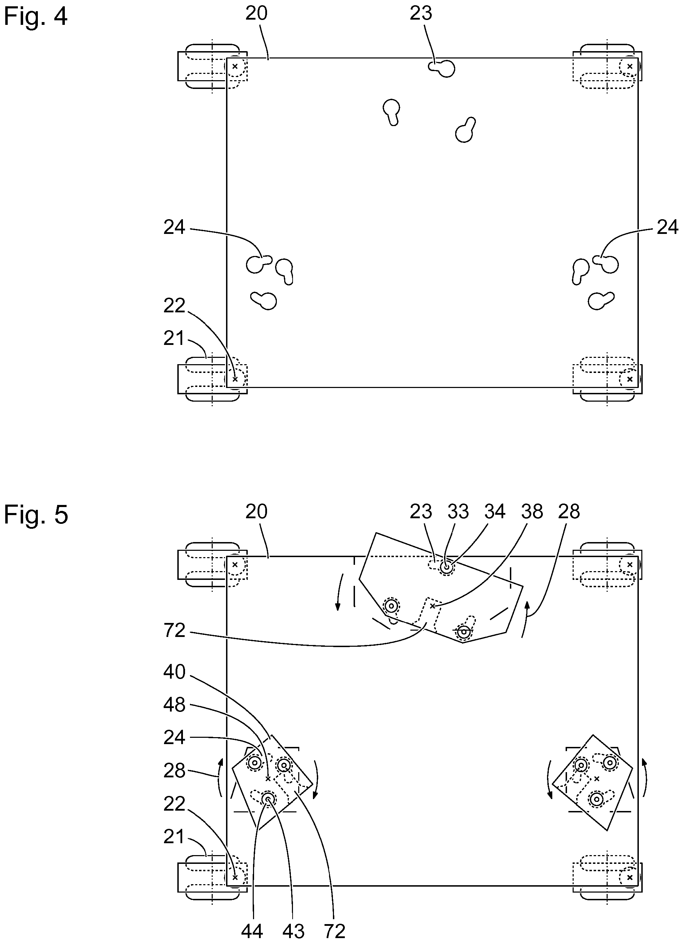

Die Befestigungsöffnungen 23, 24 weisen, wie unten anhand der Figur 4 dargestellt ist, jeweils insbesondere eine schlüssellochförmige Gestalt auf. Jeder Kopf 34, 44 einer Schraube 33, 43 kann mit einer ersten, linearen Bewegung 27 des zugeordneten Säulenmoduls 30, 40 durch einen korrespondierend breiten Abschnitt der zugeordneten Befestigungsöffnung 23, 24 hindurchgeführt werden. Bei einer nachfolgenden, unten anhand der Figur 5 dargestellten zweiten, rotatorischen Bewegung wird jede Schraube 33, 43 in einen schmalen Bereich der zugeordneten Befestigungsöffnung 23, 24 verschoben. Dadurch entstehen formschlüssige Verbindungen zwischen den Schrauben 33, 43 bzw. deren Köpfen 34, 44 und somit den dem Bodenmodul 20 zuzuwendenden Enden 32, 42 der Säulenmodule 30, 40 einerseits und dem Bodenmodul 20 andererseits.

Um das Herstellen dieser Verbindung zu vereinfachen, kann ein gewisses Spiel vorgesehen sein, so dass die Verbindung jeweils noch nicht vollkommen starr ist. Nachfolgend können durch Festziehen der Schrauben 33, 43 von der Unterseite des Bodenmoduls 20 aus die Verbindungen zwischen dem Bodenmodul 20 einerseits und den Säulenmodulen 30, 40 andererseits vervollkommnet werden.

Vor oder nach dem Festziehen der Schrauben 33, 43 zwischen dem Bodenmodul 20 und den Säulenmodulen 30, 40 kann das Brückenmodul 60 durch Schrauben 66 mit den entgegengesetzten Enden der Säulenmodule 30, 40 verbunden werden. Die Anordnung von Befestigungsöffnungen 65 im Brückenmodul 60, durch die die Schrauben 66 beim Einsetzen 67 hindurchgeführt werden, und von korrespondierenden Bohrungen 35, 45 an den dem Brückenmodul 60 zuzuwendenden Enden 36, 46 der Säulenmodule 30, 40, in die die Schrauben 66 eingeschraubt werden, stellt sicher, dass das Brückenmodul 60 mit den Säulenmodulen 30, 40 nur dann verbunden werden kann, wenn die Säulenmodule 30, 40 mit dem Bodenmodul 20 richtig verbunden sind.

Figur 4 zeigt eine schematische Darstellung des Bodenmoduls 20 des anhand der Figuren 1 bis 3 dargestellten medizinischen Gerätewagens. Die Orientierung der Zeichenebene der Figur 4 entspricht derjenigen der Figur 2.

In Figur 4 sind die schlüssellochförmigen Befestigungsöffnungen 23, 24 im Bodenmodul 20 erkennbar. Jede Befestigungsöffnung 23, 24 weist einen breiten Bereich auf, der ausgebildet ist, um ein Hindurchführen des Kopfes 34, 44 der zugeordneten Schraube 33, 43 während der ersten, linearen Bewegung 27 (vgl. Figur 3) zu ermöglichen. Jede Befestigungsöffnung 23, 24 im Bodenmodul 20 weist ferner einen schmalen Bereich auf, der insbesondere schmäler als der Kopf 34, 44 der jeweiligen Schraube 33, 43 ist, so dass eine formschlüssige Verbindung vorliegt, sobald die Schraube 33, 43 im schmalen Bereich der zugeordneten Befestigungsöffnung 23, 24 liegt.

Figur 5 zeigt eine weitere schematische Darstellung des Bodenmoduls 20 und der Säulenmodule 30, 40. Die Art der Darstellung, insbesondere die Orientierung der Zeichenebene, entspricht derjenigen der Figur 2 und 4.

In Figur 5 sind die Schrauben 33, 43 durch kleinere, die Querschnitte ihrer Schäfte andeutende Kreise und die Köpfe 34, 44 der Schrauben 33, 43 durch etwas größere Kreise dargestellt. Die Schrauben 33, 43 sind an den vom Betrachter abgewandten, nämlich dem Bodenmodul 20 zugewandten Enden 32, 42 der Säulenmodule 30, 40 (vgl. Figur 3) angeordnet und deshalb durch die Säulenmodule 30, 40 verdeckt. Trotzdem sind die Schrauben 33, 43 in Figur 5 nicht durch gestrichelte, sondern durch durchgezogene Kreise angedeutet, um sie deutlich von den Befestigungsöffnungen 23, 24 im Bodenmodul 20 zu unterscheiden.

In Figur 5 sind die Säulenmodule 30, 40 relativ zueinander und relativ zum Bodenmodul 20 so orientiert, wie dies zum Einführen der Köpfe 34, 44 der Schrauben 33, 43 durch die Befestigungsöffnungen 23, 24 bei der ersten, linearen Bewegung 27 (vgl. Figur 3) erforderlich ist. Entsprechend liegen die Köpfe 34, 44 der Schrauben 33, 43 an den Säulenmodulen 30, 40 in Deckung mit den breiten Bereichen der schlüssellochförmigen Befestigungsöffnungen 23, 34 im Bodenmodul 20.

Für jedes Säulenmodul 30, 40 ist durch Pfeile 28 eine zweite, rotatorische Bewegung, nämlich eine Rotation um eine zugeordnete Rotationsachse 38, 48 angedeutet, mit der das Säulenmodul 30, 40 in die unter anderem in Figur 2 angedeutete und in Figur 5 durch groß gestrichelte Konturen angedeutete vorgesehene Position gebracht werden kann.

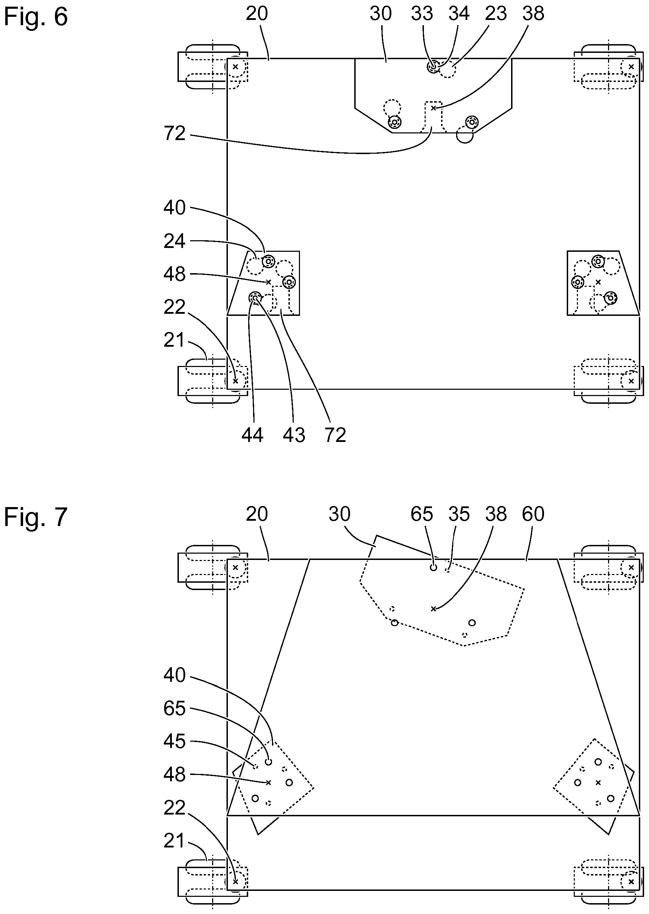

Figur 6 zeigt eine weitere schematische Darstellung des Bodenmoduls 20 und der Säulenmodule 30, 40 aus Figur 5. Die Art der Darstellung, insbesondere die Orientierung der Zeichenebene und die Darstellung der Schrauben 33, 43 und deren Köpfe 34, 44, entspricht derjenigen der Figur 5.

In Figur 6 sind die Säulenmodule 30, 40 in der nach der zweiten, rotatorischen Bewegung 28 (vgl. Figur 5) erreichten vorgesehenen Orientierung relativ zueinander und zum Bodenmodul 20 dargestellt. Die Schrauben 33, 43 durchgreifen die schmalen Bereiche der schlüssellochförmigen Befestigungsöffnungen 23, 24, so dass die Säulenmodule 30, 40 mit dem Bodenmodul 20 formschlüssig verbunden sind.

Figur 7 zeigt eine weitere schematische Darstellung des Bodenmoduls 20, der Säulenmodule 30, 40 und des Brückenmoduls 60. Die Art der Darstellung in Figur 7, insbesondere die Orientierung der Zeichenebene, entspricht derjenigen der Figuren 2 und 4 bis 6. Der Wartungsdeckel 69 (vgl. Figur 1) und entsprechende Konturen an der Oberseite des Brückenmoduls 60 sind ähnlich wie in Figur 2 nicht gezeigt.

In Figur 7 sind die Säulenmodule 30, 40 nicht in der in Figur 6 gezeigten vorgesehenen und endgültigen Orientierung, sondern in der auch in Figur 5 gezeigten Orientierung dargestellt. Bohrungen 35, 45 an den dem Brückenmodul 60 zuzuwendenden Enden 36, 46 der Säulenmodule 30, 40 (vgl. Figur 3) befinden sich nicht in Deckung mit Befestigungsöffnungen 65 im Brückenmodul 60. Deshalb kann das Brückenmodul 60 nicht mit den Säulenmodulen 30, 40 durch Schrauben 66 (vgl. Figur 3) verbunden werden.

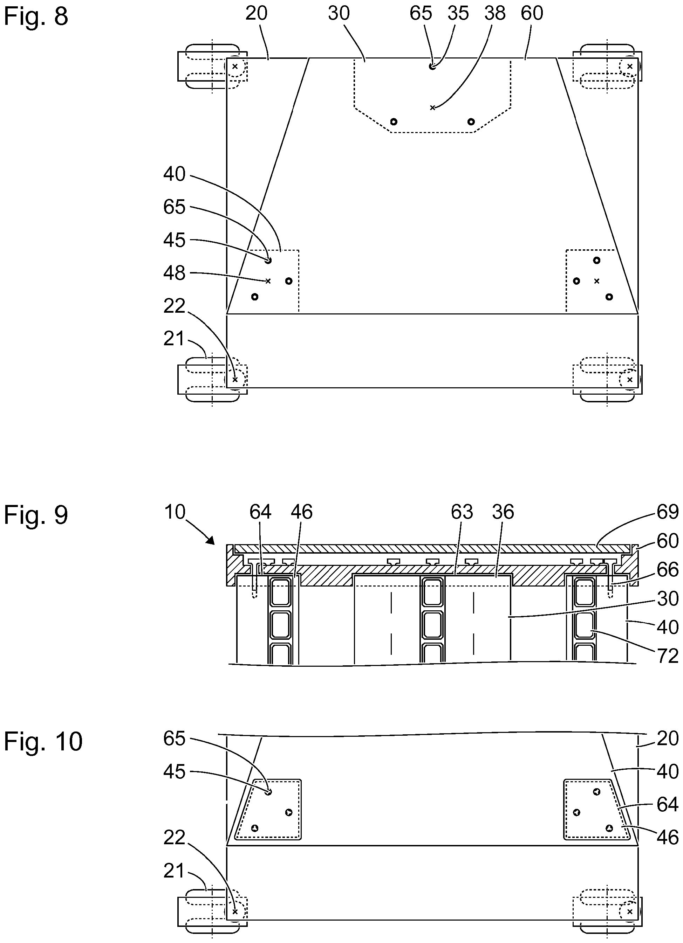

Figur 8 zeigt eine weitere schematische Darstellung des Bodenmoduls 20, der Säulenmodule 30, 40 und des Brückenmoduls 60 aus Figur 7. Die Art der Darstellung, insbesondere die Orientierung der Zeichenebene, entspricht derjenigen der Figuren 2 und 4 bis 7. Der Wartungsdeckel 69 (vgl. Figur 1) und entsprechende Konturen an der Oberseite des Brückenmoduls 60 sind ähnlich wie in den Figuren 2 und 7 nicht gezeigt.

In Figur 8 sind die Säulenmodule 30, 40 in der nach der zweiten, rotatorischen Bewegung 28 (vgl. Figur 5) erreichten, vorgesehenen und endgültigen Orientierung relativ zueinander und zum Bodenmodul dargestellt. Bohrungen 35, 45 an den dem Brückenmodul 60 zugewandten Enden 36, 46 (vgl. Figur 3) der Säulenmodule 30, 40 sind in Deckung mit Befestigungsöffnungen 65 im Brückenmodul 60. Schrauben 66 (vgl. Figur 3) können durch die Befestigungsöffnungen 65 im Brückenmodul 60 hindurch in die Bohrungen 35, 45 in den Säulenmodulen 30, 40 eingesetzt werden, um das Brückenmodul 60 mit den Säulenmodulen 30, 40 starr zu verbinden.

Der Zusammenbau des medizinischen Gerätewagens kann somit nur abgeschlossen werden, wenn die Säulenmodule 30, 40 nach der zweiten, rotatorischen Bewegung 28 (vgl. Figur 5) die vorgesehene Orientierung erreicht haben, in der sie mit dem Bodenmodul 20 formschlüssig verbunden sind.

Figur 9 zeigt eine schematische Darstellung eines Teils eines weiteren medizinischen Gerätewagens, der in einigen Merkmalen, Eigenschaften und Funktionen dem anhand der Figuren 1 bis 8 dargestellten medizinischen Gerätewagen ähnelt. Nachfolgend sind Merkmale und Eigenschaften beschrieben, in denen sich der in Figur 9 dargestellte medizinische Gerätewagen von dem anhand der Figuren 1 bis 8 dargestellten medizinischen Gerätewagen unterscheidet. Die Orientierung der Zeichenebene der Figur 9 entspricht derjenigen der Figuren 1 und 3. Im Unterschied zu den Figuren 1 und 3 ist in Figur 9 das Brückenmodul 60 wie in einem Schnitt (allerdings nicht entlang einer Ebene, sondern entlang einer Fläche, die alle drei Säulenmodule 30, 40 schneidet) dargestellt, um Ausnehmungen im Brückenmodul 60 sichtbar zu machen.

Der medizinische Gerätewagen 10 unterscheidet sich von dem anhand der Figuren 1 bis 8 dargestellten medizinischen Gerätewagen insbesondere dadurch, dass an der den Säulenmodulen 30, 40 zugewandten Unterseite des Brückenmoduls 60 Ausnehmungen 63, 64 vorgesehen sind. Position, Orientierung, Größe und Form der ersten Ausnehmung 63 im Brückenmodul 60 entsprechen denjenigen des Querschnitts des dem Brückenmodul zuzuwendenden Endes 36 des ersten Säulenmoduls 30. Position, Orientierung, Größe und Form zweier symmetrisch zueinander angeordneter zweiter Ausnehmungen 64 entsprechen denjenigen der Querschnitte der dem Brückenmodul 60 zuzuwendenden Enden 46 der zweiten Säulenmodule 40. Die zweiten Säulenmodule 40 weisen etwas kleinere Querschnitte auf als bei dem anhand der Figuren 1 bis 8 dargestellten medizinischen Gerätewagen. Wenn das Brückenmodul 60 in der vorgesehenen Weise mit den Säulenmodulen 30, 40 verbunden ist, greifen die dem Brückenmodul 60 zugewandten Enden 36, 46 in die Ausnehmungen 63, 64 an der Unterseite des Brückenmoduls 60 ein.

Figur 10 zeigt eine weitere schematische Darstellung eines Teils des medizinischen Gerätewagens aus Figur 9. Die Art der Darstellung, insbesondere die Orientierung der Zeichenebene, entspricht derjenigen der Figuren 2 und 4 bis 8. Der Wartungsdeckel 69 und entsprechende Konturen an der Oberseite des Brückenmoduls 60 sind ähnlich wie in den Figuren 7 und 8 nicht gezeigt.

In Figur 10 sind in durchgezogenen Linien die Konturen der zweiten Ausnehmungen 64 an der Unterseite des Brückenmoduls 60 und in gestrichelten Linien die äußeren Konturen der dem Brückenmodul 60 zuzuwendenden Enden der zweiten Säulenmodule 40 angedeutet. Die Querschnitte der Ausnehmungen 64 an der Unterseite des Brückenmoduls 60 einerseits und der dem Brückenmodul 60 zugewandten Enden 46 der Säulenmodule 30, 40 entsprechen einander vollständig oder weitgehend. Somit wird formschlüssig sichergestellt, dass das Brückenmodul 60 nur dann auf die Säulenmodule 30, 40 aufgesetzt werden kann, wenn diese relativ zueinander und relativ zum Bodenmodul 20 die vorgesehene Orientierung aufweisen, bei der die Säulenmodule 30, 40 formschlüssig mit dem Bodenmodul 60 verbunden sind (vgl. Figur 6 und Beschreibung dazu).

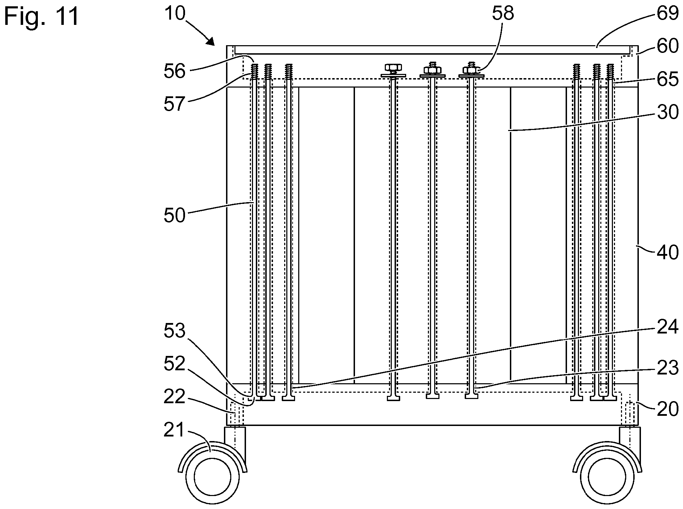

Figur 11 zeigt eine schematische Darstellung eines erfindungsgemäßen medizinischen Gerätewagens 10, der in einigen Merkmalen, Eigenschaften und Funktionen den anhand der Figuren 1 bis 10 dargestellten medizinischen Gerätewagen entspricht. Die Art der Darstellung, insbesondere die Orientierung der Zeichenebene, entspricht derjenigen der Figuren 1 und 9. Nachfolgend sind Merkmale und Eigenschaften beschrieben, in denen der medizinische Gerätewagen 10 sich von dem anhand der Figuren 1 bis 10 dargestellten medizinischen Gerätewagen unterscheidet.

Bei dem in Figur 11 dargestellten medizinischen Gerätewagen sind Befestigungsöffnungen 23, 24 im Bodenmodul 20 und Befestigungsöffnungen 65 im Brückenmodul 60 vorgesehen, wie sie anhand der Figuren 2 bis 8 dargestellt sind. Anstelle der Schrauben 33, 43, 66 bei den anhand der Figuren 1 bis 10 dargestellten medizinischen Gerätewagen, sind Zugstangen 50 in den Säulenmodulen 30, 40 vorgesehen. Jede Zugstande 50 weist an ihrem dem Bodenmodul 20 zuzuwendenden Enden 52 einen Kopf 53 auf. Der Kopf 53 ist insbesondere pilzförmig und kann dem Kopf einer Schraube ähneln. Jede Zugstange 50 weist an ihrem dem Brückenmodul 60 zuzuwendenden Ende 56 ein Außengewinde 57 auf. Jede Zugstange 50 ist in einem zugeordneten Kanal, der sich von dem Bodenmodul 20 zuzuwendenden Ende 32, 42 bis zu dem Brückenmodul 60 zuzuwendenden Ende 36, 46 erstreckt, in dem zugeordneten Säulenmodul 30, 40 angeordnet. Durch in Figur 11 nicht dargestellte Maßnahmen können die Zugstangen 50 in den Säulenmodulen 30, 40 gehalten sein, so dass sie insbesondere während Transport und Zusammenbau des medizinischen Gerätewagens nicht herausfallen können, jedoch in ihrer Längsrichtung jeweils innerhalb eines vorbestimmten Bereichs verschiebbar sind. Dabei stehen die Köpfe 53 gegenüber den dem Bodenmodul 20 zuzuwendenden Enden 32, 42 der Säulenmodule 30, 40 ähnlich wie die Köpfe 34, 44 der Schrauben 33, 43 bei dem anhand der Figur 3 dargestellten Beispiel über.

Nach dem Verbinden der Säulenmodule 30, 40 durch eine erste, lineare Bewegung 27 (vgl. Figur 3) und eine nachfolgende zweite, rotatorische Bewegung 28 (vgl. Figur 5) kann, wenn dabei die vorgesehene Orientierung der Säulenmodule 30, 40 erreicht ist, das Brückenmodul aufgesetzt werden. Dabei werden die dem Brückenmodul 60 zugewandten Enden 56 der Zugstangen 50, insbesondere die dort angeordneten Gewinde 57, durch die Befestigungsöffnungen 65 im Brückenmodul 60 hindurchgeführt. Durch Aufsetzen und Festziehen von Schraubenmuttern 58 auf den Gewinden 57 an den Zugstangen 50 können die Säulenmodule 30, 40 gleichzeitig mit dem Bodenmodul 20 und mit dem Brückenmodul 60 dauerhaft und starr verbunden werden.

### Bezugszeichen

- 10: Medizinischer Geräteträger

- 20: Bodenmodul (erstes Flächenmodul) des medizinischen Geräteträger 10
- 21: Rolleneinheit am Bodenmodul 20
- 22: vertikale Schwenkachse der Rolleneinheit 21 (Lenkbarkeit)
- 23: Befestigungsöffnung (erste Befestigungseinrichtung) im Bodenmodul 20 für erstes Säulenmodul 30
- 24: Befestigungsöffnung (erste Befestigungseinrichtung) im Bodenmodul 20 für zweites Säulenmodul 40

- 27: erste, lineare Bewegung des Säulenmoduls 30 relativ zum Bodenmodul 20
- 28: zweite, rotatorische Bewegung des Säulenmoduls 30 relativ zum Bodenmodul 20

- 30: erstes Säulenmodul des medizinischen Geräteträgers 10 (hinten Mitte)
- 32: dem Bodenmodul 20 zuzuwendendes Ende des ersten Säulenmoduls 30
- 33: Schraube (zweite Befestigungseinrichtung) am ersten Säulenmodul 30
- 34: Kopf der Schraube 33
- 35: Bohrung mit Innengewinde am ersten Säulenmodul 30
- 36: dem Brückenmodul 60 zuzuwendendes Ende des ersten Säulenmoduls 30
- 38: Rotationsachse der rotatorischen Bewegung 28

- 40: zweites Säulenmodul des medizinischen Geräteträger 10 (vorn links, rechts, baugleich spiegelsymmetrisch angeordnet)
- 42: dem Bodenmodul 20 zuzuwendendes Ende des zweiten Säulenmoduls 40
- 43: Schraube (zweite Befestigungseinrichtung) am zweiten Säulenmodul 40
- 44: Kopf der Schraube 43
- 45: Bohrung mit Innengewinde am zweiten Säulenmodul 40
- 46: dem Brückenmodul 60 zuzuwendendes Ende des zweiten Säulenmoduls 40
- 48: Rotationsachse der rotatorischen Bewegung 28
- 50: Zugstange
- 52: dem Bodenmodul zuzuwendendes Ende der Zugstange 50
- 53: Kopf an dem dem Bodenmodul 20 zuzuwendenden Ende 52 der Zugstange 50
- 56: dem Brückenmodul zuzuwendendes Ende der Zugstange 50
- 57: Gewinde an dem dem Brückenmodul 60 zuzuwendenden Ende 56 der Zugstange 50
- 58: Schraubenmutter mit zum Gewinde 57 an der Zugstange 50 korrespondierendem Gewinde

- 60: Brückenmodul (zweites Flächenmodul)
- 63: Ausnehmung im Brückenmodul 60 für erstes Säulenmodul 30
- 64: Ausnehmung im Brückenmodul 60 für zweites Säulenmodul 40
- 65: Befestigungsöffnung im Brückenmodul 60 für Zugstange 50 oder für Schraube 66
- 66: Schraube zur Verbindung zwischen Brückenmoduls 60 und Säulenmodul 30, 40
- 67: Einsetzen der Schrauben 66
- 69: Wartungsdeckel

- 72: Öffnung im Säulenmodul 30, 40

- 80: Fachboden
- 81: Eingreifhaken am Fachboden 80
- 89: Stift am Fachboden 80 zum Eingreifen in eine Öffnung 72

## Patentansprüche

1. Geräteträger (10), mit: einem Flächenmodul (20) mit einer ersten Befestigungseinrichtung (23, 24), einem Säulenmodul (30, 40) mit einer zweiten Befestigungseinrichtung (33, 43); wobei die erste Befestigungseinrichtung (23, 24) am Flächenmodul (20) und die zweite Befestigungseinrichtung (33, 43) am Säulenmodul (30, 40) so zueinander korrespondierend ausgebildet sind, dass das Säulenmodul (30, 40) und das Flächenmodul (20) mit einer ersten, linearen Bewegung (27) und einer nachfolgenden zweiten, rotatorischen Bewegung (28) des Säulenmoduls (30, 40) relativ zum Flächenmodul (20) miteinander verbindbar sind, und
einem weiteren Säulenmodul (30, 40), das mit dem Flächenmodul (20) auf gleiche Weise verbindbar ist, wie das Säulenmodul (30, 40)
wobei,
einem weiteren Flächenmodul (60), das mit den Säulenmodulen (30, 40) verbindbar ist, wobei die Säulenmodule (30, 40) und das weitere Flächenmodul (60) derart ausgebildet sind, dass das weitere Flächenmodul (60) nur dann mit den Säulenmodulen (30, 40) verbindbar ist, wenn die Säulenmodule (30, 40) relativ zueinander so positioniert und orientiert sind, wie im Fall ihrer vorgesehenen Verbindung mit dem Flächenmodul (20) wobei,
Ausnehmungen (63, 64) im weiteren Flächenmodul (60) zum Aufnehmen je eines dem weiteren Flächenmodul (60) zuzuwendenden Endes (36, 46) je eines Säulenmoduls (30, 40), wobei die Ausnehmungen (63, 64) derart angeordnet und ausgebildet sind, dass die dem weiteren Flächenmodul (60) zuzuwendenden Enden (36, 46) der Säulenmodule (30, 40) nur dann gleichzeitig in je eine zugeordnete Ausnehmung (63, 64) einführbar sind, wenn die Säulenmodule (30, 40) relativ zueinander so positioniert und orientiert sind, wie im Fall ihrer vorgesehenen Verbindung mit dem Flächenmodul (20)
**Dadurch gekennzeichnet, dass** bei dem die Befestigungseinrichtungen (23, 24, 33, 43) so ausgebildet und angeordnet sind, dass die erste, lineare Bewegung (27) nur bei einer vorbestimmten Anordnung und Orientierung des Säulenmoduls (30, 40) relativ zum Flächenmodul (20) möglich ist und bei dem Bohrungen (35, 45) so an den dem weiteren Flächenmodul (60) zuzuwendenden Enden (36, 46) der Säulenmodule (30, 40) angeordnet sind und Befestigungsöffnungen (65) so am weiteren Flächenmodul (60) angeordnet sind, dass die Bohrungen (35, 45) nur dann mit den Befestigungsöffnungen (65) zur Deckung gebracht werden können, wenn die Säulenmodule (30, 40) relativ zueinander so positioniert und orientiert sind, wie im Fall ihrer vorgesehenen Verbindung mit dem Flächenmodul (20)
wobei, bei dem die erste Befestigungseinrichtung eine Befestigungsöffnung (23, 24) am Flächenmodul (20) umfasst, die zweite Befestigungseinrichtung eine Zugstange (50) am Säulenmodul (30, 40) umfasst, die Zugstange (50) einen Kopf (53) an dem Flächenmodul (20) zuzuwendenden Ende (32, 42) des Säulenmoduls (30, 40) und ein Gewinde (57) an dem weiteren Flächenmodul (60) zuzuwendenden Ende (36, 46) des Säulenmoduls (30, 40) umfasst, der Kopf (53) der Zugstange (50) dafür vorgesehen und ausgebildet ist, gegenüber dem Flächenmodul (20) zuzuwendenden Ende (32, 42) des Säulenmodul (30, 40) überzustehen und in eine Befestigungsöffnung (23, 24) am Flächenmodul (20) einzugreifen, das Gewinde (57) der Zugstange (50) dafür vorgesehen und ausgebildet ist, gegenüber dem weiteren Flächenmodul (60) zuzuwendenden Ende (36, 46) des Säulenmoduls (30, 40) überzustehen und in eine Befestigungsöffnung (65) am weiteren Flächenmodul (60) einzugreifen.

2. Geräteträger (10) nach dem vorangehenden Anspruch, bei dem die Befestigungsöffnungen (23, 24) am Flächenmodul (20) jeweils schlüssellochförmig ausgebildet sind.

3. Geräteträger (10) nach dem vorangehenden Anspruch, bei dem: die Befestigungsöffnung (65) am weiteren Flächenmodul (60) derart angeordnet ist, dass nur dann das Gewinde (57) an der Zugstange (50) in eine Befestigungsöffnung (65) in dem weiteren Flächenmodul (60) einführbar ist, wenn die Säulenmodule (30, 40) relativ zueinander so positioniert und orientiert sind, wie im Fall ihrer vorgesehenen Verbindung mit dem Flächenmodul (20).

4. Geräteträger (10) nach einem der vorangehenden Ansprüche, ferner mit: Rädern (21) zum Ableiten der Gewichtskraft des Geräteträgers (10) in einen Boden, auf dem der Geräteträger (10) steht, wobei die Räder (21) eine reibungsarme Verschiebung des Geräteträgers (10) auf dem Boden ermöglichen.

## Claims

1. Equipment carrier (10) having: a surface module (20) with a first securing device (23, 24), a column module (30, 40) with a second securing device (33, 43); wherein the first securing device (23, 24) on the surface module (20) and the second securing device (33, 43) on the column module (30, 40) are designed corresponding to each other such that the column module (30, 40) and the surface module (20) are connectable to each other with a first, linear movement (27) and a subsequent second, rotational movement (28) of the column module (30, 40) relative to the surface module (20), and
a further column module (30, 40), which is connectable to the surface module (20) in the same way as the column module (30, 40)
wherein,
a further surface module (60), which is connectable to the column modules (30, 40), wherein the column modules (30, 40) and the further surface module (60) are designed in such a way that the further surface module (60) is connectable to the column modules (30, 40) only when the column modules (30, 40) are positioned and oriented relative to each other as in the case of their intended connection to the surface module (20) wherein,
recesses (63, 64) in the further surface module (60) for receiving a respective end (36, 46) of a respective column module (30, 40) to be directed towards the further surface module (60), wherein the recesses (63, 64) are arranged and designed in such a way that the ends (36, 46) of the column modules (30, 40) to be directed towards the further surface module (60) can be simultaneously inserted into a respective associated recess (63, 64) only when the column modules (30, 40) are positioned and oriented relative to each other as in the case of their intended connection to the surface module (20)
**characterized in that**, in which the securing devices (23, 24, 33, 43) are designed and arranged such that the first, linear movement (27) is possible only in a predetermined arrangement and orientation of the column module (30, 40) relative to the surface module (20) and
in which bores (35, 45) are so arranged at the ends (36, 46) of the column modules (30, 40) to be directed towards the further surface module (60), and securing openings (65) are so arranged on the further surface module (60), that the bores (35, 45) can be brought into coincidence with the securing openings (65) only when the column modules (30, 40) are positioned and oriented relative to each other as in the case of their intended connection to the surface module (20)
wherein, in which the first securing device comprises a securing opening (23, 24) on the surface module (20), the second securing device comprises a pull rod (50) on the column module (30, 40), the pull rod (50) comprises a head (53), at the end (32, 42) of the column module (30, 40) to be directed towards the surface module (20), and a thread (57), at the end (36, 46) of the column module (30, 40) to be directed towards the further surface module (60), the head (53) of the pull rod (50) is provided and designed to protrude in relation to the end (32, 42) of the column module (30, 40) to be directed towards the surface module (20) and to engage in a securing opening (23, 24) on the surface module (20), the thread (57) of the pull rod (50) is provided and designed to protrude in relation to the end (36, 46) of the column module (30, 40) to be directed towards the further surface module (60) and to engage in a securing opening (65) on the further surface module (60).

2. Equipment carrier (10) according to the preceding claim, in which the securing openings (23, 24) on the surface module (20) are each designed in the shape of a keyhole.

3. Equipment carrier (10) according to the preceding claim, in which: the securing opening (65) is arranged on the further surface module (60) in such a way that the thread (57) on the pull rod (50) can be inserted into a securing opening (65) in the further surface module (60) only when the column modules (30, 40) are positioned and oriented relative to each other as in the case of their intended connection to the surface module (20).

4. Equipment carrier (10) according to one of the preceding claims, furthermore having: wheels (21) for conveying the weight of the equipment carrier (10) into a floor on which the equipment carrier (10) stands, wherein the wheels (21) permit low-friction movement of the equipment carrier (10) on the floor.

## Revendications

1. Servante d'outillage (10), munie : d'un module de surface (20) muni d'un premier dispositif de fixation (23, 24), d'un module de colonne (30, 40) muni d'un deuxième dispositif de fixation (33, 43) ; le premier dispositif de fixation (23, 24) étant configuré sur le module de surface (20) et le deuxième dispositif de fixation (33, 43) étant configuré sur le module de colonne (30, 40) en correspondance l'un avec l'autre de telle sorte que le module de colonne (30, 40) et le module de surface (20) puisse être reliés l'un avec l'autre avec un premier mouvement linéaire (27) et un deuxième mouvement de rotation ultérieur (28) du module de colonne (30, 40) par rapport au module de surface (20), et
d'un module de colonne supplémentaire (30, 40), qui peut être relié avec le module de surface (20) de la même manière que le module de colonne (30, 40),
où
un module de surface supplémentaire (60), qui peut être relié avec les modules de colonne (30, 40), les modules de colonne (30, 40) et le module de surface supplémentaire (60) étant configurés de telle sorte que le module de surface supplémentaire (60) ne puisse être relié avec les modules de colonne (30, 40) que lorsque les modules de colonne (30, 40) sont positionnés et orientés l'un par rapport à l'autre comme dans le cas de leur liaison prévue avec le module de surface (20), où
des évidements (63, 64) dans le module de surface supplémentaire (60) pour la réception respectivement d'une extrémité (36, 46) d'un module de colonne (30, 40) devant être tournée vers le module de surface supplémentaire (60), les évidements (63, 64) étant agencés et configurés de telle sorte que les extrémités (36, 46) des modules de colonnes (30, 40) devant être tournées vers le module de surface supplémentaire (60) ne puissent être insérées simultanément dans un évidement associé respectif (63, 64) que lorsque les modules de colonne (30, 40) sont positionnés et orientés l'un par rapport à l'autre comme dans le cas de leur liaison prévue avec le module de surface (20), **caractérisée en ce que** dans laquelle les dispositifs de fixation (23, 24, 33, 43) sont configurés et agencés de telle sorte que le premier mouvement linéaire (27) ne soit possible qu'à un agencement et une orientation prédéterminés du module de colonne (30, 40) par rapport au module de surface (20), et
dans laquelle des alésages (35, 45) sont agencés sur les extrémités (36, 46) des modules de colonne (30, 40) devant être tournées vers le module de surface supplémentaire (60) et des ouvertures de fixation (65) sont agencées sur le module de surface supplémentaire (60), de telle sorte que les alésages (35, 45) ne puissent être recouverts avec les ouvertures de fixation (65) que lorsque les modules de colonne (30, 40) sont positionnés et orientés l'un par rapport à l'autre comme dans le cas de leur liaison prévue avec le module de surface (20),
où, dans laquelle le premier dispositif de fixation comprend une ouverture de fixation (23, 24) sur le module de surface (20), le deuxième dispositif de fixation comprend une barre de traction (50) sur le module de colonne (30, 40), la barre de traction (50) comprend une tête (53) sur les extrémités (32, 42) du module de colonne (30, 40) devant être tournées vers le module de surface (20) et un filetage (57) sur les extrémités (36, 46) du module de colonne (30, 40) devant être tournées vers le module de surface supplémentaire (60), la tête (53) de la barre de traction (50) est prévue et configurée pour dépasser par rapport aux extrémités (32, 42) du module de colonne (30, 40) devant être tournées vers le module de surface (20) et pénétrer dans une ouverture de fixation (23, 24) sur le module de surface (20), le filetage (57) de la barre de traction (50) est prévu et configuré pour dépasser par rapport aux extrémités (36, 46) du module de colonne (30, 40) devant être tournées vers le module de surface supplémentaire (60) et pénétrer dans une ouverture de fixation (65) sur le module de surface supplémentaire (60).

2. Servante d'outillage (10) selon la revendication précédente, dans laquelle les ouvertures de fixation (23, 24) sur le module de surface (20) sont à chaque fois configurées en forme de trou de serrure.

3. Servante d'outillage (10) selon la revendication précédente, dans laquelle : l'ouverture de fixation (65) sur le module de surface supplémentaire (60) est agencée de telle sorte que le filetage (57) sur la barre de traction (50) ne puisse être inséré dans une ouverture de fixation (65) dans le module de surface supplémentaire (60) que lorsque les modules de colonne (30, 40) sont positionnés et orientés l'un par rapport à l'autre comme dans le cas de leur liaison prévue avec le module de surface (20).

4. Servante d'outillage (10) selon l'une quelconque des revendications précédentes, en outre munie : de roues (21) pour la dissipation du poids de la servante d'outillage (10) dans un sol, sur lequel la servante d'outillage (10) repose, les roues (21) permettant un déplacement avec peu de frottement de la servante d'outillage (10) sur le sol.
